# EUROPÄISCHE PATENTSCHRIFT

(11) **EP 3 250 156 B1**
(45) Veröffentlichungstag und Bekanntmachung des Hinweises auf die Patenterteilung: **03.07.2019**
(21) Anmeldenummer: 16712192.0
(22) Anmeldetag: 22.01.2016
(51) Int. Cl.: A61F 2/50, C08J 7/04, C09D 183/04

(54) **VERFAHREN ZUR HERSTELLUNG EINES DAUERHAFT KOLORIERTEN GEGENSTANDS MIT EINER SILIKONOBERFLÄCHE UND NACH DEM VERFAHREN HERGESTELLTER KOLORIERTER GEGENSTAND**
METHOD FOR PRODUCING A PERMANENTLY COLORED OBJECT HAVING A SILICONE SURFACE AND COLORED OBJECT PRODUCED ACCORDING TO THE METHOD
PROCÉDÉ DE PRODUCTION D'UN ARTICLE À COLORATION STABLE DOTÉ D'UNE SURFACE SILICONE ET ARTICLE COLORÉ OBTENU D'APRÈS LEDIT PROCÉDÉ

(30) Priorität: 27.01.2015 DE 102015001075
(43) Veröffentlichungstag der Anmeldung: 06.12.2017
(73) Patentinhaber: Ottobock SE & Co. KGaA, 37115 Duderstadt (DE)
(72) Erfinder: ANHALT, Klaus-Peter, 37434 Rhumspringe (DE); ZUTHER, Martina, 37120 Bovenden (DE)
(74) Vertreter: Lins, Martina
(86) Internationale Anmeldenummer: PCT/DE2016/100026
(87) Internationale Veröffentlichungsnummer: WO 2016/119777

(56) Entgegenhaltungen:
- EP-A2- 0 985 388
- WO-A1-2014/085913

## Beschreibung

Die Erfindung betrifft ein Verfahren zur Herstellung eines dauerhaft kolorierten Gegenstands mit einer Silikonoberfläche.

Die Erfindung betrifft ferner einen dauerhaft mit Farbpigmenten kolorierten Gegenstand mit einer Silikonoberfläche.

Es ist bekannt, dass ein Gegenstand mit einer Silikonoberfläche, insbesondere ein vollständig aus Silikon bestehender Gegenstand, schwierig zu kolorieren ist, weil das Silikon die Eigenschaft hat, Fremdstoffe an der Oberfläche nicht aufzunehmen. Etwaige Farbbeschichtungen der Silikonoberfläche sind aufgrund ihrer schwachen Verbindung zur Silikonoberfläche nicht dauerhaft, wenn sie einer mechanischen Beanspruchung unterliegen.

Das Kolorierungsproblem besteht insbesondere für sogenannte Kosmetiküberzüge von Prothesen, mit denen die Hautstruktur und Hautfarbe, ggf. unter Einschluss von Finger- oder Fußnägeln möglichst naturgetreu nachgeahmt werden soll. Die Nachahmung der Hautstruktur setzt feinste Falten und Strukturen in der Silikonoberfläche voraus, die durch eine aufgebrachte Farbschicht nicht überdeckt werden dürfen.

Es ist bekannt, eine Silikonoberfläche vorzubehandeln, beispielsweise durch eine Plasmabehandlung, eine Coronaentladung durch Fluorierung oder mittels eines Primers. Eine Farbschicht darf dann nur extrem dünn aufgetragen werden, weil sie andernfalls die mühsam und zum Teil kunstvoll hergestellte Oberflächenstruktur der Silikonoberfläche überdecken würde. Eine derartige Farbschicht ist jedoch nicht ausreichend belastbar.

WO 2014/085913 A1 offenbart die Herstellung einer Prothese durch eine Anfertigung einer der Form des amputierten Glieds entsprechenden Negativform, in der eine Außenhülle dadurch hergestellt wird, dass mehrfach ein Silikonmaterial in die Form eingefüllt und wieder ausgegossen wird, sodass durch mehrere derartige Schritte mehrere Schichten einer prothetischen Hülle ausbilden. Auf diese Weise wird ein Silikon-Grundkörper hergestellt, der anschließend detailliert in herkömmlicher Weise koloriert werden kann, sodass für diese Kolorierung die oben erwähnten Nachteile auftreten.

Es ist ferner bekannt, einen derartigen Silikongegenstand, wie beispielsweise einen Silikonhandschuh zur Nachahmung einer natürlichen Handoberfläche, aus einem glasklaren durchsichtigen Silikon herzustellen und nach einer entsprechenden Vorbehandlung auf der Innenseite des Handschuhs zu kolorieren. Hierfür wird der Handschuh auf links gedreht, sodass seine Innenseite außen liegt und koloriert werden kann. Nach dem Kolorieren wird die kolorierte Außenseite wieder nach innen gestülpt, sodass die Kolorierung innen vorliegt und gegenüber einer externen Beanspruchung durch die durchsichtige Silikonschicht geschützt ist. Dieses Verfahren ist sehr aufwändig und ermöglicht keine Korrektur eines Farbtons, einer fehlerhaften Kolorierung durch eine missglückte Bemalung usw. Darüber hinaus ist es nicht möglich, nachträglich eine Farbschicht auf das vernetzte (ausvulkanisierte) Silikon aufzubringen, da hierfür wieder eine Vorbehandlung der Silikonoberfläche durchgeführt werden müsste.

Der Erfindung liegt daher die Aufgabe zugrunde, eine Kolorierung eines Gegenstands mit einer Silikonoberfläche zu ermöglichen, die dauerhaft haltbar und einfach aufzubringen ist.

Zur Lösung dieser Aufgabe ist ein Verfahren der eingangs erwähnten Art erfindungsgemäß gekennzeichnet durch die Verfahrensschritte:
a) Mischen von farbgebenden Substanzen mit einem noch vernetzungsfähigen Silikon zu einer Auftragsmischung
b) Aufquellen der Silikonoberfläche durch ein Lösemittel
c) Aufbringen der Auftragsmischung auf die Silikonoberfläche
d) Durchführung der Vernetzung des in die Silikonoberfläche eindringenden Silikons der Auftragsmischung.

Ggf. kann vor der Durchführung der Vernetzung ein Trocknungsschritt durchgeführt werden.

Das erfindungsgemäße Verfahren beruht darauf, dass die Silikonoberfläche durch das Lösemittel aufquillt und dadurch farbgebende Substanzen mit dem vernetzungsfähigen Silikon der Auftragsmischung etwas in die Silikonoberfläche eindringt. Beim anschließenden Trocknen und Vernetzen bildet sich die Quellung der Oberfläche zurück, sodass die farbgebenden Substanzen nun in einer dünnen Schicht der Silikonoberfläche verankert sind, und zwar eingebettet in Silikonteilchen, die durch die Vernetzung des in der Auftragsmischung vorhandenen Silikons entstanden sind. Das ursprünglich aufgetragene vernetzungsfähige Silikon verbindet sich mit der aufgequollenen Silikonoberfläche fest, sodass die farbgebenden Substanzen aus der Oberfläche nicht mehr ausgewaschen oder ausgerieben werden können. Erfindungsgemäß wird somit Silikonmaterial auf die aufgequollene Silikonoberfläche reaktiv aufgetragen, sodass sich durch das Ausreagieren (Vernetzen) des Silikons eine fest verankerte Farbschicht bildet.

Es wird somit eine dauerhafte und abriebfeste Kolorierung der Silikonoberfläche erreicht. Es ist ohne weiteres ersichtlich, dass eine Strukturierung der Oberfläche durch das erfindungsgemäße Verfahren erhalten bleibt, wenn die Auftragsmischung so aufgebracht und vernetzt wird, dass sie in die gequollene Silikonoberfläche eindringt und keine die Oberfläche abdeckende zusammenhängende Schicht oder nur eine sehr dünne derartige Schicht bildet.

Das die Verankerung der farbgebenden Substanzen in der Silikonoberfläche bewirkende vernetzungsfähige Silikon der Auftragsmischung kann das gleiche Silikon sein, aus dem die vernetzte Silikonoberfläche besteht. Dies ist jedoch nicht zwingend. Es ist lediglich erforderlich, eine solche Silikontype auszuwählen, die mit der Silikonoberfläche verträglich, also eine feste Verbindung mit der vernetzten Silikonoberfläche bei dem eigenen Vernetzungsvorgang eingeht. Dies ist auch bei unterschiedlichen Silikontypen ganz überwiegend der Fall. Der Fachmann verfügt über die Kenntnis der miteinander verträglichen Silikonarten.

Es ist bevorzugt, wenn das Lösemittel, das zum Aufquellen der Silikonoberfläche dient, auch als Bestandteil der Auftragsmischung verwendet wird. Dabei ist es möglich, dass das Lösemittel zum Aufquellen der Silikonoberfläche erst als Bestandteil der Auftragsmischung auf die Oberfläche aufgebracht wird, sodass die oben erwähnten Verfahrensschritte b) und c) gemeinsam durchgeführt werden, wodurch mit dem Aufbringen der Auftragsmischung erstmalig das Lösemittel auf die Silikonoberfläche gelangt. Das Aufbringen der Auftragsmischung erfolgt somit ohne eine aktivierende Vorbehandlung der Silikonoberfläche. Diese wird üblicherweise vor der Durchführung des erfindungsgemäßen Verfahrens lediglich gereinigt und von Fetten, Ölen und anderen störenden Bestandteilen befreit. Eine weitere Vorhandlung ist nicht erforderlich.

Die Vernetzung des Silikons der Auftragsmischung erfolgt nach dem Aufbringen der Auftragsmischung und dem ggf. durchführten Trocknungsschritt unter erhöhter Temperatur, also vorzugsweise in einem Ofen.

Die Auftragsmischung kann in jedem beliebigen Auftragsverfahren aufgebracht werden. Insbesondere für Kosmetiküberzüge, wie Silikonhandschuhe oder Kosmetik-Fußteile, eignet sich insbesondere das Bemalen oder Besprühen der Silikonoberfläche mit der Auftragsmischung.

Die farbgebenden Substanzen oder Farbmittel können unlösliche oder lösliche Substanzen sein, also (unlösliche) Farbpigmente oder (lösliche) Farbstoffe. Die verwendeten Farbmittel können anorganisch oder organisch sein.

Der Farbeindruck einer Kolorierung hängt einerseits von dem jeweils verwendeten Farbmittel, andererseits aber auch von der Menge des Farbmittels in einem Oberflächenbereich ab, also von der Dicke der Farbmittelschicht. Wenn diese Schicht durch Abrieb verringert wird, kann sich die Kolorierung in ihrer Intensität ändern, also mit dem Abrieb verblassen. In einer Weiterbildung der vorliegenden Erfindung wird dies dadurch verhindert, dass nach dem Aufbringen der reaktiven Farb-Silikonschicht die entstandene Oberfläche in einem aufgequollenen Zustand erneut mit einer reaktiven Silikonschicht versehen wird, die nunmehr durchsichtig ist, insbesondere farblos durchsichtig. Durch das reaktive Aufbringen der durchsichtigen Silikonschicht entsteht somit eine Abdeckung der Kolorierung, die die Kolorierung aufgrund der Durchsichtigkeit unabhängig von ihrer Dicke nicht beeinträchtigt. Wird die durchsichtige Schicht daher im Gebrauch etwas abgerieben, wird sich ihre Dicke verringern, dadurch jedoch keine sichtbare Auswirkung auf den durch die Kolorierung hervorgerufenen Farbeindruck erzeugt.

Das Aufbringen der durchsichtigen Silikonschutzschicht erfolgt mit den gleichen Schritten wie das Aufbringen der Silikon-Farbschicht für die Herstellung der Kolorierung. Für die feste Verankerung der Silikonschutzschicht wird die durch das Aufbringen der Silikon-Farbschicht gebildete Oberfläche mit einem Lösemittel aufgequollen und das Silikon der Silikon-Schutzschicht in einem noch vernetzungsfähigen Zustand aufgebracht. Die Silikon-Schutzschicht bildet sich somit ebenfalls durch eine in-situ-Vernetzung des Silikons zu der Silikonschutzschicht. Wie für das Aufbringen der Silikon-Farbschicht kann das Lösemittel vorab auf die Oberfläche aufgebracht werden oder in einem einstufigen Verfahren mit der entsprechenden reaktiven Auftragsmischung.

Die oben erwähnte Aufgabe wird ferner gelöst durch einen Gegenstand mit einer Silikonoberfläche, der durch Aufbringen einer farbgebende Substanzen enthaltenden Farbschicht auf die Silikonoberfläche koloriert ist, bei dem die Farbpigmente bis zu einer geringen Tiefe von größenordnungsmäßig 1 µm in eine aufgequollene Silikonoberfläche eingedrungen sind und eine durch das Vernetzen des Silikons fest verankerte Farbschicht bilden. Dabei ist die Silikonoberfläche vorzugsweise frei von einer sie abdeckenden zusammenhängenden Schicht.

Der dauerhaft kolorierte Gegenstand kann aus einem beliebigen Material bestehen und eine Silikonoberfläche aufweisen. Bevorzugt ist jedoch ein Gegenstand, der vollständig aus Silikon besteht, wie dies beispielsweise für einen bevorzugten Gegenstand in Form eines Kosmetiküberzugs für eine Prothese der Fall sein kann. Mit der Beschreibung, dass der Gegenstand vollständig aus Silikon besteht, soll jedoch nicht ausgeschlossen werden, dass der Gegenstand auf einer der Silikonoberfläche abgewandten Oberfläche noch eine Funktionsbeschichtung aufweist, beispielsweise eine die Gleitfähigkeit erhöhende Beschichtung, die aus einem Textil oder einer geeigneten Kunststoffschicht, beispielsweise aus Parylen (Poly (para-xylylen)) bestehen kann.

Wie oben erläutert worden ist, kann der kolorierte Gegenstand insbesondere eine transparente Abdeckschicht aus Silikon oberhalb der Kolorierung aufweisen, die als Schutzschicht für die Kolorierung fungiert. Denkbar ist ferner, die transparente Schutzschicht aus einem anderen Material auszubilden, das fest mit dem kolorierten Silikon verbunden werden kann. Diese Ausführungsform ließe sich beispielsweise mit einer transparenten Schicht aus Parylen realisieren, die durch eine CVD-Beschichtung (Chemical Vapor Deposition) auf die Silikonoberfläche aufgebracht werden kann.

Die erfindungsgemäße Kolorierung der Silikonoberfläche funktioniert auch bei vollständig vernetzten Silikonen, selbst bei Silikonen die über ein Jahr oder mehr gelagert worden sind, und zwar ohne vorherige aktivierende Vorbehandlung, wie Primern, Plasmabehandlung, Coronaentladung, Fluorierung usw. Bereits verwendetes Silikon muss lediglich von etwaigen Verschmutzungen gründlich gereinigt werden und ist dann zur Kolorierung nach dem erfindungsgemäßen Verfahren geeignet.

### Beispiel 1 Silikonhandschuh (Handflächen außen/innen)

Ein Silikonhandschuh wird von aufgrund elektrostatischer Aufladung anhaftenden Staubpartikeln und Fusseln mit 634A58 Isopropanol gereinigt. Dazu wird ein sauberes, fusselfreies Tuch oder Textil verwendet. Es wird eine Trocknungszeit von 10 min bei Raumtemperatur vorgesehen, weil durch die Verdunstungskälte des Isopropanols Wasser aus der Luftfeuchtigkeit auf der Oberfläche kondensieren kann, das ebenfalls wieder verdunstet sein muss.

Die verwendeten Materialien sind

| Feststoff | Silikon | LSR 3003-50 | 100,00% |
|---|---|---|---|
| Lösungsmittel | Silikonfluid | Q7-9180 | + 278,08% |
| Farbe | Silikonfarbe | Elastosil | |
| | | | |
| | Weiß | RAL 9010 | + 2,27% |
| | Dunkelblau | RAL 5010 | +3,63 % |
| | Dunkelrot | RAL3000 | + 6,09% |
| | Rot | RAL3020 | + 0,69% |

Bemerkung: Lösungsmittel und Farbe sind prozentual auf den Feststoffgehalt berechnet.

Mit der Silikonfarbe Elastosil wird somit eine weiße, eine dunkelblaue, eine dunkelrote und eine rote Auftragsmischung hergestellt, und zwar zusammen mit dem Silikonfluid als Lösungsmittel.

Das verwendete Material für den Silikonhandschuh ist hier als Feststoff-Silikon bezeichnet.

Von der hergestellten und im Kühlschrank aufbewahrten Farben werden ca. 5 ml entnommen und auf einen nicht saugfähigen Untergrund (Glas- oder Keramikplatte) gegeben. Weitere ca. 7 ml reines Lösemittel (Silikonfluid) wird daneben gegeben. Ein Synthetikpinsel wird mit dem gleichen Lösemittel benetzt und dann eine kleine Menge der vorbereiteten Farbmenge von der Platte mit dem Pinsel abgenommen und nach Bedarf mit extra Lösemittel verdünnt, um die Farbintensität zu variieren und die Trocknungszeit zu verlängern. Zusätzlich wird hierdurch wie gewünscht eine unscharfe Farbgrenze erzielt.

Eine gewünschte Mattheit des Auftrags kann durch Abtupfen mit einem feinporigen Schwamm o. ä. während der Trocknungsphase, in der die Silikonfarbe nicht mehr fließt, aber noch nicht hart ist, beeinflusst werden.

### Beispiel 2 Bemalung von Nägeln

Der Silikonhandschuh wird von aufgrund elektrostatischer Aufladung anhaftender Staubpartikel und Fussel mit 634A5 Kaltreiniger (aliphatisches Kohlenwasserstoffgemisch Fraktion 185°C) gereinigt. Hierzu wird ein sauberes, fusselfreies Tuch oder Textil verwendet. Nach einer Trocknungszeit von 5 min kann die Auftragsmischung aufgebracht werden. Die verwendeten Materialien sind

| Feststoff | Silikon | LSR 3003-50 | 100,00 % |
|---|---|---|---|
| Lösungsmittel | Kaltreiniger | | + 278,08% |
| Farbe | Silikonfarbe | Elastosil | |
| | | | |
| | Weiß | RAL 9010 | + 34,67% |
| | Schwarz | RAL 9011 | + 0,79% |
| | Dunkelblau | RAL 5010 | + 1,76% |
| | Gelb | RAL 1016 | + 5,29% |
| | Gelb | RAL 1021 | + 1,26% |
| | Dunkelrot | RAL 3000 | + 0,20% |

Bemerkung: Lösungsmittel und Farbe sind prozentual auf den Feststoffgehalt berechnet.

Von der hergestellten und im Kühlschrank aufbewahrten Farbmischung wird ca. 2 ml entnommen und auf einen nicht saugfähigen Untergrund (Glas- oder Keramikplatte) gegeben. Die Silikonfarbe Elastosil (Wacker Chemie, München) ist eine auf Silikon basierte Pigmentzusammensetzung.

Dann wird eine kleine Menge der vorbereiteten Farbmischung von der Platte mit einem Schlepper-Synthetikpinsel Größe 5/0 aufgenommen und damit direkt auf das Silikon im Nagelbereich gemalt.

Für die Bemalung des Nagelmondes und der Nagelspitze ist ein deckender Strich mit klarer Farbgrenze nötig. Dieser wird erreicht, wenn rezepturgemäß ein hoher Pigmentanteil und ein niedriger Lösemittelanteil vorliegen. Dadurch wird es erforderlich, die Rezeptur durch die Art des verwendeten Lösemittels langsam verdunstend einzustellen, damit genügend Zeit zum glatten Verstreichen bleibt. Dies könnte auch mit einem höheren Lösemittel eines schnell verdunstenden Systems erreicht werden, durch den hohen Lösemittelanteil könnte jedoch nicht die Farbintensität erreicht werden.

### Beispiel 3 Spravtechnik

Der Silikonhandschuh wird von aufgrund elektrostatischer Aufladung anhaftenden Staubpartikeln oder Fusseln mit 634A81 Silikonfluid gereinigt. Dazu wird ein sauberes, fusselfreies Tuch oder Textil verwendet. Nach einer Trocknungszeit von 10 min (wie im Beispiel 1) kann die Auftragsmischung aufgesprüht werden. Hierzu werden Nagelbereiche mit Nagellack o. ä. abgedeckt, um ein Besprühen mit der Farbe zu verhindern. Die verwendeten Materialien sind

| Feststoff | Silikon | LSR 3003-50 | 100,00% |
|---|---|---|---|
| Lösungsmittel | Silikonfluid | Q7-9180 | + 600,00% |
| Farbe | Silikonfarbe | Elastosil | |
| | | | |
| | Gelb | RAL 1021 | + 10,00% |
| | Dunkelrot | RAL 3000 | + 5,83% |
| | Schwarz | RAL 9011 | + 12,71% |
| Mattierungsmittel | Kieselsäure | Acematt 3300® | + 1,89% |

| | | | |
|---|---|---|---|
| * Hersteller: Evonik Industries AG, Essen | | | |

Bemerkung: Lösungsmittel, Farbe und Mattierungsmittel sind prozentual auf den Feststoffgehalt berechnet.

Die Farbmischung wird in eine Sprühpistole bzw. ein Airbrushsystem gegeben und mit zwei bar Druck in mehreren Durchgängen auf die Silikonoberfläche aufgesprüht. Der Schutzlack im Nagelbereich wird anschließend mit Nagellackentferner wieder abgelöst und anschließend wird die Bemalung im Nagelbereich gemäß Beispiel 2 durchgeführt.

Nach dem Bemalen oder Besprühen kann der Gegenstand 10 bis 30 min bei Raumtemperatur trocknen und dann im Ofen bei 120°C für vier Stunden oder 150°C für zwei Stunden ausvulkanisieren/vernetzen. Dieses gilt für alle Beispiele 1 bis 3.

## Patentansprüche

1. Verfahren zur Herstellung eines dauerhaft kolorierten Gegenstands mit einer Silikonoberfläche, **gekennzeichnet durch** die Verfahrensschritte:
a) Mischen von farbgebenden Substanzen mit einem noch vernetzungsfähigen Silikon zu einer Auftragsmischung
b) Aufquellen der Silikonoberfläche durch ein Lösemittel
c) Aufbringen der Auftragsmischung auf die Silikonoberfläche
d) Durchführung der Vernetzung des in die Silikonoberfläche eindringenden Silikons der Auftragsmischung

2. Verfahren nach Anspruch 1, **gekennzeichnet durch** einen Trocknungsschritt vor der Durchführung der Vernetzung.

3. Verfahren nach Anspruch 1 oder 2, **dadurch gekennzeichnet, dass** die Auftragsmischung mit dem die Silikonoberfläche aufquellenden Lösemittel hergestellt wird.

4. Verfahren nach Anspruch 1, **dadurch gekennzeichnet, dass** das Lösemittel zum Aufquellen der Silikonoberfläche als Bestandteil der Auftragsmischung auf die Silikonoberfläche aufgebracht wird.

5. Verfahren nach einem der Ansprüche 1 bis 4, **dadurch gekennzeichnet, dass** das Aufbringen der Auftragsmischung ohne eine aktivierende Vorbehandlung der Silikonoberfläche erfolgt.

6. Verfahren nach einem der Ansprüche 1 bis 5, **dadurch gekennzeichnet, dass** die Vernetzung des Silikons der Auftragsmischung unter erhöhter Temperatur erfolgt.

7. Verfahren nach einem der Ansprüche 1 bis 6, **dadurch gekennzeichnet, dass** die Auftragsmischung so aufgebracht und vernetzt wird, dass sie in die gequollene Silikonoberfläche eindringt und keine die Oberfläche abdeckende zusammenhängende Schicht bildet.

8. Verfahren nach einem der Ansprüche 1 bis 7, **dadurch gekennzeichnet, dass** die Auftragsmischung durch Bemalen oder Besprühen aufgebracht wird.

9. Verfahren nach einem der Ansprüche 1 bis 8, **dadurch gekennzeichnet, dass** während der Vernetzung der den Farbstoff enthaltenden Auftragsmischung auf die so erhaltene Oberfläche im aufgequollenen Zustand eine ein vernetzungsfähiges Silikon enthaltende durchsichtige Schicht aufgebracht wird und die Vernetzung des in die aufgequollene Oberfläche eingedrungenen Silikons durchgeführt wird.

10. Verfahren nach einem der Ansprüche 1 bis 8, **dadurch gekennzeichnet, dass** nach der Vernetzung der den Farbstoff enthaltenden Auftragsmischung die so erhaltene Oberfläche durch ein Lösungsmittel aufgequollen wird und im aufgequollenen Zustand eine ein vernetzungsfähiges Silikon enthaltende durchsichtige Schicht aufgebracht wird und die Vernetzung des in die aufgequollene Oberfläche eingedrungenen Silikons durchgeführt wird.

11. Gegenstand mit einer Silikonoberfläche, der durch Aufbringen einer farbgebende Substanzen enthaltenden Farbschicht auf die Silikonoberfläche koloriert ist, **dadurch gekennzeichnet, dass** die farbgebenden Substanzen nach Eindringen in die aufgequollene Silikonoberfläche eine durch das Vernetzen des Silikons fest verankerte Farbschicht bilden.

12. Gegenstand nach Anspruch 11, **dadurch gekennzeichnet, dass** die Silikonoberfläche frei von einer sie abdeckenden zusammenhängenden Schicht ist.

13. Gegenstand nach Anspruch 11 oder 12, **dadurch gekennzeichnet, dass** auf die Silikonoberfläche eine durchsichtige Abdeckschicht aufgebracht ist.

14. Gegenstand nach Anspruch 13, **dadurch gekennzeichnet, dass** die durchsichtige Abdeckschicht aus Silikon besteht.

15. Gegenstand nach einem der Ansprüche 11 bis 14 in Form eines Kosmetiküberzugs für eine Prothese.

16. Gegenstand nach einem der Ansprüche 11 bis 15, **dadurch gekennzeichnet, dass** er vollständig aus Silikon besteht.

## Claims

1. A method for manufacturing a permanently colored article having a silicone surface, said method being **characterized by** the steps of:
a) mixing color-conferring substances with a still crosslinkable silicone to form an application mixture,
b) solvent swelling the silicone surface,
c) applying the application mixture to the silicone surface,
d) crosslinking the application mixture silicone which penetrates into the silicone surface.

2. The method as claimed in claim 1, **characterized by** a drying step before the crosslinking step.

3. The method as claimed in claim 1 or 2, **characterized in that** the application mixture is formed with the solvent used to swell the silicone surface.

4. The method as claimed in claim 1, **characterized in that** the solvent for swelling the silicone surface is applied to the silicone surface as a constituent part of the application mixture.

5. The method as claimed in any one of claims 1 to 4, **characterized in that** the step of applying the application mixture is effected without any activating pretreatment of the silicone surface.

6. The method as claimed in any one of claims 1 to 5, **characterized in that** the step of crosslinking the silicone of the application mixture is effected under elevated temperature.

7. The method as claimed in any one of claims 1 to 6, **characterized in that** the application mixture is both applied and crosslinked so as to penetrate into the swollen silicone surface and not form a coherent layer covering the surface.

8. The method as claimed in any one of claims 1 to 7, **characterized in that** the application mixture is applied by painting or spraying.

9. The method as claimed in any one of claims 1 to 8, **characterized in that** during the step of crosslinking the application mixture, which comprises the dye, the surface thus obtained has applied to it, in its swollen state, a transparent layer comprising a crosslinkable silicone and the step of crosslinking the silicone which has penetrated into the swollen surface is effected.

10. The method as claimed in any one of claims 1 to 8, **characterized in that** after the step of crosslinking the application mixture, which comprises the dye, the surface thus obtained is swollen by a solvent and has applied to it, in its swollen state, a transparent layer comprising a crosslinkable silicone and the step of crosslinking the silicone which has penetrated into the swollen surface is effected.

11. An article having a silicone surface which is colored by applying a color layer comprising color-conferring substances to the silicone surface, **characterized in that** the color-conferring substances penetrate into the swollen silicone surface to form a firmly attached layer of color as the silicone crosslinks.

12. The article as claimed in claim 11, **characterized in that** the silicone surface is free from any coherent layer covering it.

13. The article as claimed in claim 11 or 12, **characterized in that** a transparent covering layer has been applied to the silicone surface.

14. The article as claimed in claim 13, **characterized in that** the transparent covering layer consists of silicone.

15. The article as claimed in any one of claims 11 to 14 in the form of a cosmetic cover for a prosthesis.

16. The article as claimed in any one of claims 11 to 15, **characterized in that** it consists entirely of silicone.

## Revendications

1. Procédé de production d'un objet à coloration permanente doté d'une surface en silicone, caractérisé en par les étapes consistant à :
a) mélanger des substances colorantes avec une silicone encore réticulable pour donner un mélange à appliquer,
b) faire gonfler la surface silicone par un solvant,
c) appliquer le mélange à appliquer sur la surface silicone,
d) mettre en oeuvre la réticulation de la silicone, pénétrant dans la surface silicone, du mélange à appliquer.

2. Procédé selon la revendication 1,
**caractérisé par** une étape de séchage avant de mettre en oeuvre la réticulation.

3. Procédé selon la revendication 1 ou 2,
**caractérisé en ce que** le mélange à appliquer est préparé avec le solvant faisant gonfler la surface silicone.

4. Procédé selon la revendication 1,
**caractérisé en ce que** le solvant pour faire gonfler la surface silicone est appliqué à titre de composante du mélange à appliquer sur la surface silicone.

5. Procédé selon l'une des revendications 1 à 4,
**caractérisé en ce que** l'application du mélange à appliquer s'effectue sans prétraitement d'activation de la surface silicone.

6. Procédé selon l'une des revendications 1 à 5,
**caractérisé en ce que** la réticulation de la silicone du mélange à appliquer s'effectue à température accrue.

7. Procédé selon l'une des revendications 1 à 6,
**caractérisé en ce que** le mélange à appliquer est appliqué et réticulé de manière à pénétrer dans la surface silicone gonflée et à ne pas former de couche cohérente recouvrant la surface.

8. Procédé selon l'une des revendications 1 à 7,
**caractérisé en ce que** le mélange à appliquer est appliqué par peinture ou par pulvérisation.

9. Procédé selon l'une des revendications 1 à 8,
**caractérisé en ce que**
pendant la réticulation du mélange à appliquer contenant le colorant, une couche transparente contenant une silicone réticulable est appliquée sur la surface ainsi obtenue, dans l'état gonflé, et la réticulation de la silicone se fait lorsqu'elle a pénétré dans la surface gonflée.

10. Procédé selon l'une des revendications 1 à 8,
**caractérisé en ce qu'**après la réticulation du mélange à appliquer contenant le colorant, la surface ainsi obtenue est amenée à gonfler à l'aide d'un solvant, et dans l'état gonflé une couche transparente contenant une silicone réticulable est appliquée, et la réticulation de la silicone se fait lorsqu'elle a pénétré dans la surface gonflée.

11. Objet doté d'une surface silicone qui est coloré par application d'une couche de couleur contenant des substances colorantes sur la surface silicone,
**caractérisé en ce que** les substances colorantes constituent, après pénétration dans la surface silicone gonflée, une couche de couleur fermement ancrée par la réticulation de la silicone.

12. Objet selon la revendication 11,
**caractérisé en ce que**
la surface silicone est dépourvue d'une couche cohérente la recouvrant.

13. Objet selon la revendication 11 ou 12,
**caractérisé en ce qu'**une couche de recouvrement transparente est appliquée sur la surface silicone.

14. Objet selon la revendication 13,
**caractérisé en ce que** la couche de recouvrement transparente est constituée en silicone.

15. Objet selon l'une des revendications 11 à 14 sous la forme d'un revêtement esthétique pour une prothèse.

16. Objet selon l'une des revendications 11 à 15,
**caractérisé en ce qu'**il est constitué complètement en silicone.
